# EUROPEAN PATENT SPECIFICATION

(11) **EP 1 029 536 B1**
(45) Date of publication and mention of the grant of the patent: **28.11.2007**
(21) Application number: 00109347.5
(22) Date of filing: 01.10.1997
(51) Int. Cl.: A61K 9/00, A61K 9/12

(54) **Buccal non-polar spray**
Nichtpolare Spray zur bukkalen Verabreichung
Pulverisateur buccal non polaire

(43) Date of publication of application: 23.08.2000
(62) Divisional of application: 97911621.7
(73) Proprietor: Novadel Pharma Inc., Flemington, NJ 08822 (US)
(72) Inventor: Dugger, Harry A., Flemington, NJ 08822 (US)
(74) Representative: Luderschmidt, Schüler & Partner

(56) References cited:
- EP-A- 0 504 112
- WO-A-97/38663
- WO-A-97/38687
- DE-A- 4 038 203
- DE-A- 4 132 176

## Description

### BACKGROUND OF THE INVENTION

It is known that certain biologically active compounds are better absorbed through the oral mucosa than through other routes of administration, such as through the stomach or intestine. However, formulations suitable for such administration by these latter routes present their own problems. For example, the biologically active compound must be compatible with the other components of the composition such as propellants, solvents, etc. Many such formulations have been proposed. For example, U.S.P. 4,689,233. Dvorsky et al., describes a soft gelatin capsule for the administration of the anti-coronary drug nifedipine dissolved in a mixture of polyether alcohols. U.S.P. 4,755,389, Jones et al., describes a hard gelatin chewable capsule containing nifedipine. A chewable gelatin capsule containing a solution or dispersion of a drug is described in U.S.P. 4,935,243, Borkan et al. U.S.P. 4,919,919, Aouda et al, and U.S.P. 5,370,862, Klokkers-Bethke, describe a nitroglycerin spray for administration to the oral mucosa comprising nitroglycerin, ethanol, and other components. An orally administered pump spray is described by Cholcha in U.S.P. 5,186,925. Aerosol compositions containing a hydrocarbon propellant and a drug for administration to a mucosal surface are described in U.K. 2,082,457, Su, U.S.P. 3,155,574, Silson et al., U.S.P. 5,011,678, Wang et al., and by Parnell in U.S.P. 5,128,132. It should be noted that these references discuss bioavailability of solutions by inhalation rather than through the membranes to which they are administered.

### SUMMARY OF THE INVENTION

A buccal aerosol spray using a non-polar solvent has now been developed which provides biologically active compounds for rapid absorption through the oral mucosa, resulting in fast onset of effect.

The buccal aerosol spray compositions of the present invention, for transmucosal administration of a pharmacologically active compound soluble in a pharmacologically acceptable non-polar solvent comprising in weight % of total composition: pharmaceutically acceptable propellant 5-80%, non-polar solvent 20-85%, active compound 0.05-50%, suitably additionally comprising, by weight of total composition a flavoring agent 0.01-10%. Preferably the composition comprises: propellant 10-80%, non-polar solvent 25-85%, active compound 0.05-40%, flavoring agent 1-8%; most suitably propellant 20-70%, non-polar solvent 30-74.75%, active compound 0.25-35%, flavoring agent 2-7.5%.

It is an object of the invention to coat the mucosal membranes with extremely fine droplets of spray containing the active compounds.

It is also an object of the invention to administer to a mammalian in need of same preferably man, a predetermined amount of a biologically active compound by this method.

The non-polar spray compositions are intended to be administered from a sealed aerosol spray container containing a composition of the non polar spray formulation, and a metered valve suitable for releasing from said container a predetermined amount of said composition.

As the propellant evaporates after activation of the aerosol valve, a mist of fine droplets is formed which contains solvent and active compound.

The propellant is a non-Freon material, a C₃₋₈ hydrocarbon of a linear or branched configuration. The propellant should be substantially non-aqueous. The propellant produces a pressure in the aerosol container such that under expected normal usage it will produce sufficient pressure to expel the solvent from the container when the valve is activated but not excessive pressure such as to damage the container or valve seals.

The non-polar solvent is a non-polar hydrocarbon, (C₂-C₂₄) fatty acid C₂-C₆ esters, C₇-C₁₈ hydrocarbon, C₂-C₆ alkanoyl esters, and the triglycerides of the corresponding acids, preferably a C₇₋₁₈ hydrocarbon of a linear or branched configuration, fatty acid esters, and triglycerides, such as miglyol. The solvent must dissolve the active compound and be miscible with the propellant, i.e., solvent and propellant must form a single phase at 0-40°C at a pressure range of 1-3 atm.

The non-polar aerosol spray compositions of the invention are intended to be administered from a sealed, pressurized container. Unlike a pump spray, which allows the entry of air into the container after every activation, the aerosol container of the invention is sealed at the time of manufacture. The contents of the container are released by activation of a metered valve, will does not allow entry of atmospheric gasses with each activation. Such containers are commercially available.

The active compound may include biologically active peptides, sulfonyl ureas, antibiotics, antifungals, antivirals, sleep inducers, antiemetics, histamine H-2 receptor antagonists, barbiturates, prostaglandins; terbutaline, and theophylline, provided that the composition of the invention does not comprise weight % of total composition propellant 50-80%, non-polar solvent 20-50%, benzodiazepines 0,05-15%.

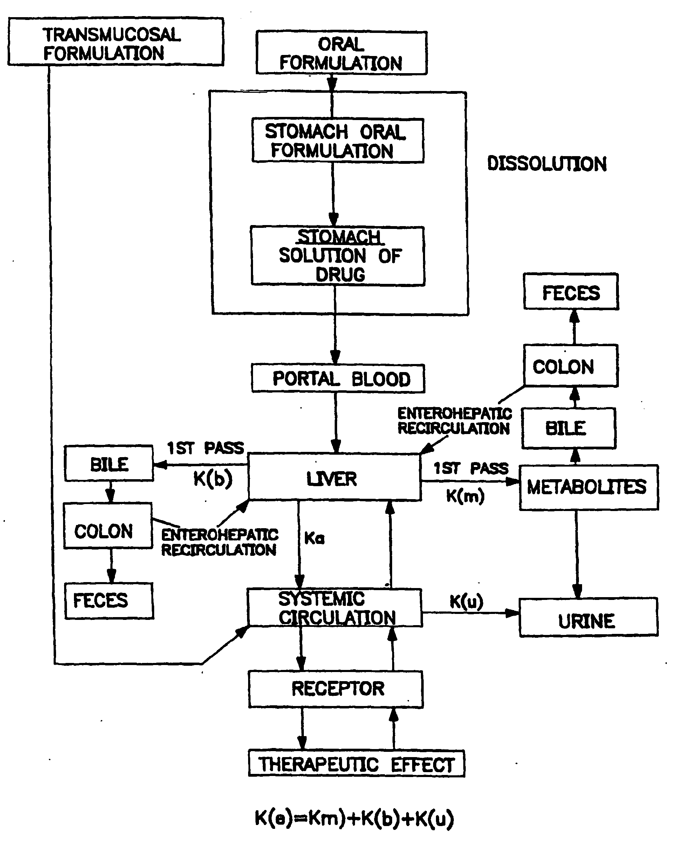

### BRIEF DESCRIPTION OF THE DRAWING

The figure is a schematic diagram showing routes of absorption and processing of pharmacologically active substances in a mammalian system.

### DESCRIPTION OF THE PREFERRED EMBODIMENTS

The preferred active compounds of the present invention are in anionized, salt form or as the free base of the pharmaceutically acceptable salts thereof (provided, they are soluble in the spray solvent). These compounds are soluble in the non-polar solvents of the invention at useful concentrations. These concentrations may be less than the standard accepted dose for these compounds since there is enhanced absorption of the compounds through the oral mucosa. This aspect of the invention is especially important when there is a large (40-99.99%) First pass effect.

As propellants for the non polar sprays, propane, N-butane, iso-butane, N-pentane, iso-pentane, and neo-pentane, and mixtures thereof may be used. N-butane and iso-butane, as single gases, are the preferred propellants. It is permissible for the propellant to have a water content of no more than 0.2%, typically 0.1-0.2%. (All percentages herein are by weight unless otherwise indicated.) It is also preferable that the propellant be synthetically produced to minimize the presence of contaminants which are harmful to the active compounds. These contaminants include oxidizing agents, reducing agents, Lewis acids or bases, and water. The concentration of each of these should be less than 0.1%, except that water may be as high as 0.2%.

The non-polar solvents for the non-polar sprays are selected from the group consisting of (C₂-C₂₄) fatty acid C₂-C₆ esters, C₇-C₁₈ hydrocarbon, C₂-C₆ alkanoyl esters, and the triglycerides of the corresponding acids.

The preferred flavoring agents are synthetic or natural oil of peppermint, oil of spearmint, citrus oil, fruit flavors, sweeteners (sugars, aspartame, saccharin, etc.), and combinations thereof.

The active substances include the active compounds selected from the group consisting of cyclosporine, sermorelin, Octreotide acetate, calcitonin-salmon, insulin lispro, glyburide, zidovudine, erythromycin, ciprofloxacin, ondansetron hydrochloride, dimenhydrinate, cimetidine hydrochloride, famotidine, phenytoin sodium, phenytoin, carboprost thromethamine, carboprost, diphenhydramine hydrochloride, terbutaline sulfate, terbutaline, theophylline, and the like.

The formulations of the present invention comprise an active compound or a pharmaceutically acceptable salt thereof. The term "pharmaceuticaily acceptable salts" refers to salts prepared from pharmaceutically acceptable non-toxic acids or bases including organic and inorganic acids or bases.

When an active compound of the present invention is acidic, salts may be prepared from pharmaceutically acceptable non-toxic bases. Salts derived from all stable forms of inorganic bases include aluminum, ammonium, calcium, copper, iron, lithium, magnesium, manganese, potassium, sodium, zinc, etc. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines and basic ionexchange resins such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, isopropylamine, lysine, methyl-glucosamine, morpholine, piperazine, piperidine, polyamine resins, procaine, purine, theobromine, triethylamine, trimethylamine, tripropylamine, etc.

When an active compound of the present invention is basic, salts may be prepared from pharmaceutically acceptable non-toxic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, mandelic, methanesulfonic, mucic, nitric, pamoic, pantothenic, phosphoric, succinic, sulfuric, tartaric, p-toluenesulfonic, etc. Particularly preferred are citric, hydrobromic, maleic, phosphoric, sulfuric, and tartaric acids.

In the discussion of methods of treatment herein, reference to the active compounds is meant to also include the pharmaceutically acceptable salts thereof. While certain formulations are set forth herein, the actual amounts to be administered to the mammal or man in need of same are to be determined by the treating physician.

The invention is further defined by reference to the following examples, which are intended to be illustrative and not limiting.

### EXAMPLE 4

### Antibiotics anti-fungals and anti-virals

| A. zidovudine formerly called azidothymidine (AZT) (Ratrovir) non-polar lingual spray | | | |
|---|---|---|---|
| | Amounts | preferred amount | most preferred amount |
| zidovudine | 10-50 | 15-40 | 25-35 |
| Soya oil | 20-85 | 25-70 | 30-40 |
| Butane | 15-80 | 30-75 | 60-70 |
| flavors | 0.1-5 | 1-4 | 2-3 |

| C. Famotidine non-polar lingual spray | | | |
|---|---|---|---|
| | Amounts | preferred amount | most preferred amount |
| Famotidine | 1.35 | 5-30 | 7-20 |
| Soya oil | 10-50 | 15-40 | 15-20 |
| Butane | 15-80 | 30-75 | 45-70 |
| polyoxyethylated oleic glycerides | 10-50 | 15-40 | 15-20 |
| flavors | 0.1-5 | 1-4 | 2-3 |

### EXAMPLE 7

### Barbiturates

| B. Phenytoin non-polar lingual spray | | | |
|---|---|---|---|
| | Amounts | preferred amount | most preferred amount |
| Phenytoin | 5-45 | 10-40 | 15-35 |
| migylol | 10-50 | 15-40 | 15-20 |
| Butane | 15-80 | 30-75 | 60-70 |
| polyoxyethylated oleic glycerides | 10-50 | 15-40 | 15-20 |
| flavors | 0.1-10 | 1,8 | 5-7.5 |

### EXAMPLE 8

### Prostaglandins

| B. Carboprost non-polar lingual spray | | | |
|---|---|---|---|
| | Amounts | preferred amount | most preferred amount |
| Carboprost | 0.05-5 | 0.1-3 | 0.25-2.5 |
| migylol | 25-50 | 30-45 | 35-40 |
| Butane | 5-60 | 10-50 | 20-35 |
| polyoxyethylated oleic glycerides | 25-50 | 30-45 | 35-40 |
| flavors | 0.1-10 | 1-8 | 5-7.5 |

| C. Terbutaline as non-polar lingual spray | | | |
|---|---|---|---|
| | Amounts | preferred amount | most preferred amount |
| Terbutaline | 0.1-10 | 0.2-7.5 | 0.5-6 |
| migylol | 25-50 | 30-45 | 35-40 |
| isobutane | 5-60 | 10-50 | 20-35 |
| polyoxyethylated oleic glycerides | 25-50 | 30-46 | 35-40 |
| flavors | 0.110 | 1.8 | 5-7.5 |

## Claims

1. A buccal aerosol spray composition for transmucosal administration of a pharmacologically active compound,
wherein said active compound is soluble in a pharmacologically acceptable non-polar solvent, said composition comprising in weight % of total composition:
pharmaceutically acceptable propellant selected from the group consisting of C₃₋₈ hydrocarbon of a linear or branched configuration 5-80 %, non-polar solvent 20-85 %, active compound 0.05-50 %,
wherein the active compound is selected from the group consisting of biologically active peptides, sulfonyl ureas, antibiotics, antifungals, antivirals, antiemetics, histamine H-2 receptor antagonists, sleep inducers, barbiturates, prostoglandins, terbutaline, and theophylline,
wherein the solvent is selected from the group consisting of (C₂-C₂₄) fatty acid (C₂-C₆) esters, C₇-C₁₈ hydrocarbons of a linear or branched configuration, and C₂-C₆ alkanoyl esters, and triglycerides of the corresponding acids,
provided that
said composition does not comprise in weight % of total composition:
propellant 50-80 %, non-polar solvent 20-50 %, benzodiazepines 0.05-15 %.

2. The composition of claim 1 additionally comprising, by weight of total composition: flavoring agent 0.1-10%.

3. The composition of claim 1 wherein the active compound is selected from the group consisting of cyclosporin, clozapine, zidevudine, erythromycin, odansetron, cimetidine, phenytoin, and carboprost, in their nonionized form or as the pharmaceutically acceptable salts thereof.

4. The composition of claim 2 wherein the flavoring agents are selected from the group consisting of synthetic or natural oil of peppermint, oil of spearmint, citrus oil, fruit flavors, sweeteners and combinations thereof.

5. The composition of claim 1 comprising: propellant 20 - 70 %, non-polar solvent 30 - 74.75 %, active compound 0.25 - 35 %, flavoring agent 2 - 7.5 %.

6. The composition of claim 1 wherein the propellant is propane, n-butane, iso-butane, n-pentane, iso-pentane, or neo-pentane, and mixtures thereof.

7. The composition of claim 1 wherein the propellant is n-butane or iso-butane and has a water content of no more than 0.2 % and oxidizing agents, reducing agents, and Lewis acids or bases content in a concentration of less than 0.1 %.

8. The composition of claim 1 wherein the solvent comprises a triglyceride.

## Patentansprüche

1. Bukkale Aerosolspray-Zusammensetzung zur transmukosen Verabreichung einer pharmazeutisch aktiven Verbindung, wobei die aktive Verbindung in einem pharmazeutisch verträglichen, nicht-polaren Lösungsmittel löslich ist, wobei die Zusammensetzung in Gewichtsprozent der Gesamtzusammensetzung enthält:
pharmazeutisch verträgliches Treibmittel, ausgewählt aus der Gruppe, bestehend aus C₃₋₈ Kohlenwasserstoff mit einer linearen oder verzweigten Konfiguration, 5-80%, nicht-polares Lösungsmittel 20-85%, aktive Verbindung 0,05-50%,
wobei die aktive Verbindung aus der Gruppe ausgewählt ist, die aus biologisch aktiven Peptiden, Sulfonylharnstoffen, Antibiotika, Antimykotika, antiviralen Wirkstoffen, Antiemetika, Histamin H-2-Rezeptor Antagonisten, Schlafmitteln, Barbituraten, Prostoglandinen, Terbutalin und Theophyllin besteht,
wobei das Lösungsmittel aus der Gruppe ausgewählt ist, die aus (C₂-C₂₄) Fettsäure (C₂-C₆) Estern, C₇-C₁₈ Kohlenwasserstoffen einer linearen oder verzweigten Konfiguration und C₂-C₆ Alkanoylestern und Triglyceriden der entsprechenden Säuren besteht,
unter der Voraussetzung, dass
die Zusammensetzung nicht in Gewichtsprozent der Gesamtzusammensetzung enthält:
Treibmittel 50-80%, nicht-polares Lösungsmittel 20-50%, Benzodiazepine 0,05-15%.

2. Zusammensetzung nach Anspruch 1, weiterhin enthaltend in Gewichtsprozent der Gesamtzusammensetzung:
Aromastoff 0,1-10%.

3. Zusammensetzung nach Anspruch 1, wobei die aktive Verbindung aus der Gruppe ausgewählt ist, die aus Cyclosporin, Clozapin, Zidevudin, Erythromycin, Odansetron, Cimetidin, Phenytoin und Carboprost in ihrer nicht-ionisierten Form oder als pharmazeutisch verträgliche Salze davon besteht.

4. Zusammensetzung nach Anspruch 2, wobei die Aromastoffe aus der Gruppe ausgewählt sind, die aus synthetischem oder natürlichem Pfefferminzöl, Öl der grünen Minze, Zitrusöl, Fruchtaromen, Süßstoffen und Kombinationen davon besteht.

5. Zusammensetzung nach Anspruch 1, umfassend: Treibmittel 20-70%, nicht-polares Lösungsmittel 30-74,75%, aktive Verbindung 0,25-35%, Aromastoff 2-7,5%.

6. Zusammensetzung nach Anspruch 1, wobei das Treibmittel Propan, n-Butan, iso-Butan, n-Pentan, iso-Pentan oder neo-Pentan und Mischungen davon ist.

7. Zusammensetzung nach Anspruch 1, wobei das Treibmittel n-Bütan oder iso-Butan ist und einen Wassergehalt von nicht mehr 0,2% und einen Oxidationsmittel-, Reduktionsmittel- und Lewissäuren- oder Basen-Gehalt in einer Konzentration von weniger als 0,1% hat.

8. Zusammensetzung nach Anspruch 1, wobei das Lösungsmittel ein Triglycerid umfasst.

## Revendications

1. Composition de spray d'aérosol buccal pour l'administration transmuqueuse d'un composé pharmacologiquement actif,
où ledit composé actif est soluble dans un solvant non polaire pharmacologiquement acceptable, ladite composition comprenant en % en masse de la composition totale :
propulseur pharmaceutiquement acceptable choisi dans le groupe consistant en un C₃₋₈ hydrocarbure d'une configuration linéaire ou ramifiée 5-80%, solvant non polaire 20-85 %, composé actif 0,05-50 %,
où le composé actif est choisi dans le groupe consistant en les peptides biologiquement actifs, les sulfonylurées, les antibiotiques, les antifongiques, les antiviraux, les antiémétiques, les antagonistes de récepteur d'histamine H-2, les inducteurs de sommeil, les barbituriques, les prostaglandines, la terbutaline et la théophylline,
où le solvant est choisi dans le groupe consistant en les (C₂-C₆) esters de (C₂-C₂₄) acides gras, les C₇-C₁₈ hydrocarbures d'une configuration linéaire ou ramifiée, et les C₂-C₆ alcanoyl esters, et les triglycérides des acides correspondants,
à condition que
ladite composition ne comprenne pas en % en masse de la composition totale :
propulseur 50-80 %, solvant non polaire 20-50 %, benzodiazépines 0,05-15 %.

2. Composition selon la revendication 1 comprenant en outre, en masse de la composition totale :
agent aromatisant 0,1 - 10 %.

3. Composition selon la revendication 1 où le composé actif est choisi dans le groupe consistant en la cyclosporine, la clozapine, la zidévudine, l'érythromycine, l'odansetron, la cimétidine, la phénytoïne, et le carboprost, sous leur forme non ionisée ou sous forme de leurs sels pharmaceutiquement acceptables.

4. Composition selon la revendication 2 où les agents aromatisants sont choisis dans le groupe consistant en l'essence de menthe poivrée synthétique ou naturelle, l'essence de menthe verte, l'essence d'agrumes, les arômes de fruits, les édulcorants et leurs combinaisons.

5. Composition selon la revendication 1 comprenant : propulseur 20-70 %, solvant non polaire 30-74,75 %, composé actif 0,25-35 %, agent aromatisant 2-7,5 %.

6. Composition selon la revendication 1 où le propulseur est le propane, le n-butane, l'isobutane, le n-pentane, l'isopentane ou le néopentane, et leurs mélanges.

7. Composition selon la revendication 1 où le propulseur est le n-butane ou l'isobutane et a une teneur en eau ne dépassant pas 0,2 % et une teneur en agents oxydants, agents réducteurs et acides ou bases de Lewis en une concentration inférieure à 0,1 %.

8. Composition selon la revendication 1 où le solvant comprend un triglycéride.
